# EUROPEAN PATENT APPLICATION

(11) **EP 4 819 192 A1**
(43) Date of publication of application: **07.10.2026**
(21) Application number: 25167731.6
(22) Date of filing: 01.04.2025
(51) Int. Cl.: H01J 49/00, G01N 33/68

(54) **PROTOCOL FOR HOMOLOGOUS ROW IDENTIFICATION AND ANALYSIS IN LC MS/MS EXPERIMENTS**

(71) Applicant: Bruker Daltonics GmbH & Co. KG, 28359 Bremen (DE)
(72) Inventor: The designation of the inventor has not yet been filed
(74) Representative: Bremi, Tobias Hans

(57) **Abstract**

Method for the identification of molecular changes between homologous rows of analytes of a first sample and of a second sample different from the first sample, analyzed by using untargeted LC MS/MS experiments.

The method comprises the following steps:
i) identifying analytes and isotope patterns in the samples;
ii) annotating said analytes, by using a spectral library, rule-based annotation or simulations;
iii) calculating the change of intensity of each annotated analyte, preferably as the binary logarithm of such a ratio;
iv) sorting the data of the analytes as a function of said at least one repeating structural feature and thereby identifying analytes with differential behavior within homologous rows of analytes in said samples ;
v) scheduling a targeted LC MS/MS of the first and/or the second sample for the identified analytes.

## Description

### TECHNICAL FIELD

The present invention relates to a protocol for homologous row identification and/or analysis in LC MS/MS experiments, in particular in the field of lipidomics, metabolomics or proteomics, and for targeted scheduling of corresponding experiments, in particular of the PRM PASEF type.

### PRIOR ART

The motion of molecular ions in gases as compared to their motion in a condensed phase has established ion mobility as a powerful separation tool, especially in conjunction with mass spectrometry. Ion mobility spectrometry (IMS), such as drift tube ion mobility spectrometry (DTIMS), travelling wave ion mobility spectrometry (TWIMS), and differential mobility spectrometry (DMS), also known as field asymmetric ion mobility spectrometry (FAIMS), act as a post-ionization method for gas-phase filtering of ions in a controlled atmosphere, fractionating complex samples, suppressing chemical noise, separating isobaric mixtures, and characterizing molecular ions based on their mobility coefficients. These are methods that separate gas phase ions based on their interaction with a collision gas and their masses. In the first step, the ions are separated according to their mobility through a buffer gas on a millisecond timescale using an ion mobility spectrometer. The separated ions are then introduced into a mass analyzer in a second step where their mass-to-charge ratios can be determined on a microsecond timescale. The effective separation of analytes achieved with this method makes it widely applicable in the analysis of complex samples such as in proteomics, lipidomics and metabolomics.

US 7,838,826 B1 (M. A. Park, 2008) and the corresponding patent family members presents a small ion mobility analyzer/spectrometer which has become known under the acronym "TIMS" analyzer/spectrometer (TIMS = trapped ion mobility spectrometry). The terms ion mobility analyzer and ion mobility spectrometer are used interchangeably here. A TIMS analyzer comprises a gas flow that drives ions against a counter-acting electric field barrier such that the ions are at first trapped along the axis of the TIMS analyzer. The ions are confined in the radial direction by an electric RF field. After transferring ions from an ion source to the electric field barrier, the height of the electric field barrier or the gas velocity is adjusted such that ion species are released from the electric field barrier in the sequence of their mobility.

Commonly, the length of the ion mobility separation unit of a TIMS analyzer amounts to about five centimeters only. In a small tube with an inner diameter of about eight millimeters, a radial RF quadrupole field is generated to hold ions near to the axis. A gas flow inside a tube drives ions entrained in the gas flow against a ramped counter-acting electric DC field barrier where the ions are trapped and separated according to their mobilities at locations on the field ramp at which the friction force of the moving gas equals the counter-acting force of the electric DC field on the ramp. After loading the TIMS with ions, the height of the electric DC field barrier is decreased; this scan releases the ion species in the sequence of their mobility. Unlike many other trials to build small ion mobility spectrometers, the small device by M. A. Park has already achieved, with reduced scan speeds, ion mobility resolutions up to R_{mob} = 400, which is extraordinarily high.

A TIMS analyzer with parallel ion accumulation is described in US 9,683,964; it improves the utilization of the ions from the ion source to nearly 100%. Importantly, many ions sources, such as electrospray ion sources produce ions continuously. TIMS with parallel accumulation can also be operated to collect and analyze ions continuously - that is, TIMS can operate at 100% duty cycle. Barring pseudopotential or space charge effects, substantially all ions of the ion source are collected and analyzed without loss. TIMS with parallel ion accumulation further provides the unique possibility to prolong the ion accumulation duration to find more detectable ion species, thereby even increasing the ion mobility resolution by a corresponding prolongation of the scan time. The ions are collected in an accumulator unit, preferably almost identical to the scanning unit, at a ramp of an electric DC field barrier such that they get spatially separated by their ion mobility along the ramp. Therefore, the accumulated ions are less influenced by space charge than in other types of accumulator units. Of greatest importance, however, is the unique feature of a TIMS analyzer that a longer accumulation period permits to increase the mobility resolution by choosing correspondingly longer mobility scan durations, e.g. 100 milliseconds scan duration with an ion mobility resolution of R_{mob} = 75 instead of 20 milliseconds scan duration with R_{mob} = 30. As a consequence of the higher number of ions collected and the better ion mobility resolution, more ion species can be detected and measured. Once an ion mobility scan is completed (optionally after twenty to some hundred milliseconds), the accumulated ions are transferred (in about a millisecond) from the accumulation unit to the scanning unit, and the next ion mobility scan can be started. In total, a skilled practitioner will appreciate that it will be possible to achieve a measurement rate of 300 to 450 ion species per second. If TIMS with parallel ion accumulation is installed in tandem mass spectrometer (MS/MS instrument) an MS-MS instrument, 300 to 450 characteristic fragment ion spectra per second may be measured quantitatively.

WO-A-2019096852 relates to use of an isobaric label in mass spectrometry (MS) analysis using data-independent acquisition (DIA), wherein said isobaric label comprises or consists of a group which fragments in the mass spectrometer (i) at an energy below the energy required for fragmenting analyte-derived precursor ions and/or a higher conversion rate than said precursor ions; and (ii) at said energy according to (i) and when coupled to a precursor ion, at a single site within said group, to yield a first moiety and a second moiety, said second moiety being coupled to said precursor ion. It proposes the use of a trapped ion mobility spectrometry-time of flight (timsTOF) instrument, equipped with parallel/serial fragmentation (PASEF); see, e.g. Meier et al. 2015, doi: 10.1021/acs.jproteome.5b00932. US-A-2019371585 relates to selection of precursors from a measured mobility-mass map for tandem mass spectrometry and is based on processing a peak list from measured signals and clustering these peaks in the mobility-mass space.

US-A-2022034840 discloses an apparatus and a method of data independent combined ion mobility and mass spectroscopy analysis which includes introducing precursor ions into an ion mobility spectrometer (IMS), sequentially releasing precursor ions from said IMS according to their ion mobility, introducing said released precursor ions into a mass filter, fragmenting the precursor ions transmitted through said mass filter to generate fragment ions, and carrying out a mass spectroscopy measurement on said fragment ions. The IMS and mass filter are controlled in a synchronized manner to carry out a plurality of IM scans, wherein adjacent mass windows in said IM scan that are associated with consecutive mass spectroscopy measurements of fragment ions overlap, such that precursor ions transmitted through said mass filter during said IM scan are located in at least one continuous scan region in an m/z-IM plane which extends in a generally diagonal direction in said m/z-IM plane.

Targeted selection of bio sample analytes, e.g. peptides from complex protein digests, subjected to fragmentation is a performance-limiting factor in tandem mass spectrometry (MS). The problem is mainly attributed to the large number of co-eluting high abundance analytes and limitations in the duty cycle of the mass spectrometer arranged to operate in data-dependent acquisition (DDA) mode whereby precursor ions are selected sequentially for fragmentation in order of decreasing intensity. Furthermore, the depth of proteomic analyses is also limited by the overwhelming proportion of high abundance background ions that compromise the identification and quantification of low abundance analytes. Whether low-intensity precursors are selected for fragmentation is primarily dictated by the speed at which the mass analyzer can perform tandem MS analysis. The dynamic range of the analysis in DDA mode can be extended by excluding previously analyzed precursor ions, but this strategy is no longer efficient for highly complex samples with an extended dynamic range in concentration. The duty cycle of the MS remains a problem even in data-independent acquisition (DIA) mode where all ions within a selected mass-to-charge range are selected for fragmentation. The issue of sample complexity is routinely addressed by fractionating the sample.

Liquid chromatography coupled to Mass Spectrometry (LC-MS) has now been used for many years in the proteomic and lipidomic community for the identification and quantification of biological analytes, including lipids, carbohydrates, peptides (and thus proteins) and combinations thereof from complex sample mixtures. In lipidomics and proteomics, the analytes are typically lipids and peptides, respectively, and derivatives thereof, e.g. generated by tryptic digestion of protein samples or extraction of lipids e.g. using organic solvents. The commonly most used approaches are variants of the so-called LC-MS/MS or "shotgun" MS approach that is based on the generation of fragment ions from precursor ions that are automatically selected based on the precursor ion profiles (data dependent analysis, DDA). A main shortcoming of these methods is poor reproducibility which results in only partially overlapping lipid/protein sets in repeated analysis of substantially similar samples. Several new approaches have recently been developed that address these limitations and which can conceptually be described as targeted proteomics approaches.

Another variant of measurement is data independent acquisition (DIA). Contrary to MRM or PRM, this approach does not require any preliminary method design prior to the sample injection. Since the LC-MS acquisition covers the complete analyte contents of a sample through the entire mass and retention time (RT) ranges the data can be mined a posteriori for any lipid or peptide/precursor of interest. Data is acquired in a data independent manner, on the complete mass range (e.g. 200-2000 Thomson) and through the entire chromatography, disregarding of the content of the sample. This is commonly achieved by stepping the wide selection window of the mass analyzer step by step through the complete mass range. In effect, this data acquisition method generates a complete fragment ion map for all the analytes present in the sample and relates the fragment ion spectra back to the precursor ion selection window in which the fragment ion spectra were acquired. This is achieved by widening the precursor isolation windows on the mass analyzer and thus accounting a priori for multiple precursors co-eluting and concomitantly participating to the fragmentation pattern recorded during the analysis. Such a precursor window is called a precursor selection window. The result is complex fragment ion spectra from multiple precursor fragmentations, that require a more challenging data analysis.

Alton et al in https://doi.org/10.5194/egusphere-2022-1319 report, that owing to advances in instrumentation, particularly with regards to mass resolving power and instrument response factors (sensitivities), hundreds of different mass-to-charge (m/z) signals are routinely measured. This large number of detected ions creates challenges for data visualization. Furthermore, assignment of chemical formulas to these ions is time-consuming and increases in difficulty at the higher m/z ranges. They present a technique called scaled Kendrick mass defect (SKMD) analysis to facilitate the visualization and peak identification processes for typical atmospheric organic (and to some extent inorganic) compounds. SKMD is related to the previously proposed resolution enhanced Kendrick mass defect (REKMD). SKMD introduces a tunable integer scaling factor into the mass defect equation that effectively contracts or expands the mass scale. The SKMD transformation maintains the horizontal alignment of ion series related by integer multiples of the chosen base unit that is characteristic of Kendrick mass defect analysis. However, the tunable integer acts to alter the mass defect spacing between different homologue ion series. As a result, the entire mass defect range (-0.5 to 0.5) is more effectively used simplifying data visualization and facilitating chemical formula assignment. They describe the mechanism of this transformation and discuss base unit and scaling factor selections appropriate for compounds typically found in atmospheric measurements.

### SUMMARY OF THE INVENTION

Both, instrumentation and subsequent data processing have advanced so far in e.g. lipidomics and metabolomics, that the lipid content of a sample (read "lipidome" for organisms) can be assessed with a high coverage (how many lipids of how many lipid classes can be found and annotated) and with a higher specificity (how exact can the molecular lipid species or even structure be annotated). However, linking the experimental findings and data to a biological interpretation is still lacking systematic automated support and almost solely relies on manual/visual inspection.

Here, we suggest a workflow including data acquisition, over automated processing, over automated annotation, to a new statistical analysis and a new interactive tool for data exploration, that adds to biological interpretation in an untargeted fashion, i.e. without involving a preselected metabolic pathway.

Statistical approaches to perturbation e.g. in lipidomic analyses focus either on individual lipid species, which are already known to be relevant for a certain phenotype, or on high-level inspection of dynamics in lipid class abundances. In other words, samples from different conditions (conditions: may for example be wild type vs. mutant strains, or healthy vs. diseased patients, or plants from different origins) are typically compared by inspecting the abundances of individual lipid species, or the sum of abundances of lipid classes. Neither approach is particularly well suited to represent the systematic, step by step nature of lipid synthesis.

With the proposed new approach, we embrace both the classes level, but also the contribution and interconnection of individual species in such a class. For the case of lipidomics, we embrace both the lipid classes level, but also the contribution and interconnection of individual lipid species The homologous rows, which e.g. lipid species form (i.e. same number of double bond equivalents (DBE) with changing number of carbon atoms (n_{C}) in the side chains, or the same number of carbon atoms in the side chains (n_{C}) with changing number of double bond equivalents (DBE)) are focused. The patterns (or grids) formed by these homologous rows constitute a class, in case of lipidomics a lipid class. The abundances of e.g. the individual lipid species in these lipidomics patterns form a topology. While the overall abundance of a lipid class in two conditions (the fold-change) may seem unchanged, the topology within the same class may change very much: It may tilt in direction of n_{C}, DBE or both. It may form linear or higher degree slopes. It may plateau in certain areas at a specific fold-change.

As an example, a mutant strain of any organism may be uncapable of (or hampered in) desaturating (i.e. introducing DBEs) lipid species of a certain lipid class. This does not necessarily change the total lipid signal intensity in this class, but it will change the topology of the above-described pattern.

We make this perspective accessible in an (interactive) visualization, and for automated detection of topologic pattern recognition. And we present a workflow, where this initial untargeted analysis (e.g. LC-TIMS-PASEF) serves as a selection of biologically relevant features for a subsequent targeted and highly quantitative analysis (e.g. prmPASEF).

According to an example protocol for initial overall illustration of the concept, it comprises the following steps:
1. The raw data of samples of two different conditions (fold change) is acquired with LC MS/MS, preferably in a data independent manner, e.g. using LC-TIMS-PASEF;
2. The raw data can be preprocessed in an untargeted fashion, which yields a feature table. A feature comprises ions (analytes) and e.g. respective isotope patterns, aligned across all samples from step 1;
3. Rule-based analyte annotation or analyte annotation with a spectral library or any other analyte annotation approach (including simulations such as transfer learning approaches), so for lipidomics lipid annotation or lipid annotation with a spectral library or any other lipid annotation approach, applied to annotate features from step 2 with at least analyte (lipid) species level;
4. The log2(fold change) between intensities of each annotated analyte (e.g. lipid or glycosylated system) in the two conditions is calculated;
5. Sorting/and/or visualization (see also figures):
   a. e.g. a Kendrick Mass Defect plot (e.g. RT vs. KMD(CH2) or SKMD(CH2)) is used to show all analytes (e.g. lipids), or all analytes (e.g. lipids) of a certain class in a way that reflects the pattern of homologous rows;
   b. The log2(fold changes) can, if needed, used to e.g. control the representation, e.g. bubble size or the color of each lipid species in the plot;
   c. Topological features of the lipid class are thus revealed;
6. Automated analysis (see also figures):
   a. Matrices of DBEs vs. n_{C} are calculated for each analyte (e.g. lipid or glycosylated system) class;
   b. The value for each coordinate (DBE/n_{C}) can, if needed, set to the log2(fold change) of the abundances of the respective lipid species in the two conditions;
   c. The resulting topologies are classified to stereotypic topologies (stereotypic topologies may be selected knowledge-driven/manually, or by a clustering, see also the above example with the organism uncapable of desaturating) and correlation values (as a measure of confidence) are calculated;
7. Selection of candidates for targeted scheduling, e.g. prmPASEF:
   a. Based on manual selection (see step 5) and/or automated analysis (see step 6), analytes (e.g. lipids or glycosylated systems) that participate in patterns that correlate with certain topologies are selected for subsequent targeted analysis in targeted scheduling such as prmPASEF.

More generally speaking, according to a first aspect of the present invention, it relates to a method for the identification of molecular changes between homologous rows of analytes of a first sample and of a second sample different from the first sample, both samples analyzed by e.g. using untargeted LC MS/MS experiments. The two samples are different in the sense that they are the same or similar in type but of different origin or of the same origin and have been subjected to perturbation. For example, one sample can be taken from a healthy patient and another one from a diseased patient, but still being the same type of sample (e.g. a blood sample). Or one sample can be a wild type sample and another one can be a knockout sample, again being the same type of sample. Said homologous rows of analytes systematically differ by at least one repeating structural feature.

The proposed method comprises the following steps:
i) identifying analytes (typically in terms of MS1, MS2, RT, and, if measured, CCS information, including if desired isotope patterns) in the data obtained from the first sample and in the data obtained from the second sample;
ii) annotating said analytes in the data obtained from the first sample and in the data obtained from the second sample, preferably by using a spectral library, rule-based annotation or simulations, or a combination thereof;
iii) calculating the change of intensity of each annotated analyte between the two samples, preferably as a ratio, most preferably as the binary logarithm of such a ratio;
iv) sorting the data of the analytes as a function of said at least one repeating structural feature and thereby identifying analytes with differential (intensity) behavior within homologous rows of analytes in said first sample and said second sample;
v) scheduling a targeted LC MS/MS of the first and/or the second sample for the identified analytes.

The method is defined as involving two samples, however it can equally applied to comparing three samples or more samples.

### Definitions:

The mass-to-charge ratio is represented as (m/z), where (m) is the mass of the ion (typically in atomic mass units, amu) and (z) is absolute charge of the ion (which can be positive or negative depending on the measurement mode; typically and especially for proteomics one is in positive mode, having less electrons than protons in the ion if one goes for the elementary particles of the atoms; molecules are usually getting charged by attaching H⁺ so protons). This ratio is dimensionless and crucial for identifying and characterizing ion. The m/z value is calculated by dividing the mass of the ion by its charge. For example, an ion with a mass of 100 amu and a charge of +1 will have an m/z value of 100. If the same ion has a charge of +2, its m/z value will be 50. If it is the same ion it will be roughly 50.5 because of the additional proton, assumed as the charge carrier: MH+ : (99+1)/1 = 100 Da, MH2^2+: (99+2)/2 = 50.5 Da. Other formulation: If an ion with a mass of 100 amu has a charge of +2 its m/z value will be 50. In a mass spectrometer, ions are separated based on their m/z using electric and magnetic fields. This separation allows for the detection and analysis of ions with different masses and charges. The m/z values help in identifying the molecular structure and composition of the sample. By analyzing the mass spectrum, which plots ion intensity against m/z, chemists can determine the presence of specific ions and infer the structure of the molecules.

Mass spectrometry involves ionizing chemical compounds to generate charged particles (ions). These ions are then separated and detected based on their mass-to-charge ratio (m/z). The resulting data, presented as a mass spectrum, provides valuable information about the molecular structure and composition of the sample. An ionization source ionizes the sample molecules, meaning it converts them into ions by adding or removing charge carriers, typically H⁺ for lipdomics or proteomics applications with electrospray ion sources. The mass analyzer separates the ions based on the mass-to-charge ratio, and ions are detected by the detector. The instrument computer converts the detector signal into a mass spectrum. A mass spectrometer typically operates under vacuum conditions to ensure a collision-free flight path between ionization source and detector.

A mass spectrum is a plot that displays the ion signal as a function of the mass-to-charge ratio (m/z) of the ions present in a sample. This spectrum is typically generated using a mass spectrometer and provides valuable information about the molecular composition and structure of the sample. The x-axis of a mass spectrum represents the mass-to-charge ratio, while the y-axis shows the intensity of the ion signal, which correlates with the abundance of each ion. By analyzing the peaks in a mass spectrum, chemists can identify and quantify different molecules within the sample.

The Kendrick mass is defined by setting the mass of a chosen molecular fragment, e.g. CH2, to an integer value in u (unified atomic mass unit). The Kendrick mass is evaluated by multiplying the IUPAC mass scale with the factor 14.0000 u/14.01565 u = 0.9988834. The Kendrick mass defect (KMD) is defined as the exact Kendrick mass subtracted from the nominal (integer) Kendrick mass. This results in the same Kendrick mass defect for homologous components in that series. E.g., the members of an alkylation series which have the same degree of unsaturation and number of heteroatoms (nitrogen, oxygen and sulfur) but differ in the number of CH2 units have the same Kendrick mass defect. A Kendrick mass defect plot is a data representation, in which one axis is given by the Kendrick mass defect of the respective analytes. In a Kendrick mass analysis, the Kendrick mass defect is plotted as function of nominal Kendrick mass for ions observed in a mass spectrum. Ions of the same family, for example the members of an alkylation series, have the same Kendrick mass defect but different nominal Kendrick mass and are positioned along a horizontal line on the plot. If the composition of one ion in the family can be determined, the composition of the other ions can be inferred. Horizontal lines of different Kendrick mass defect correspond to ions of different composition, for example degree of saturation or heteroatom content. a. A KMD plot (e.g. RT vs. KMD(CH2)) can be used to show, for the case of a lipid analysis, all lipids, or all lipids of a certain lipid class in a way that reflects the pattern of homologous rows.

A homologous row (or equally termed homologous series) is a sequence of compounds with the same functional group and similar chemical properties in which the members of the series systematically differ by structural features such as the number of repeating units (e.g. CH2 in particular in case of lipids or sugar units in case of glycosylated systems) or the number and nature of the repeating units (e.g. double bond or not, DBE) they contain. For the case of lipids, the homologous rows are given as the group of lipid species having same number of double bond equivalents (DBE) with changing number of carbon atoms (nC) in the side chains, or the group of lipid species having the same number of carbon atoms in the side chains (nC) with changing number of double bond equivalents (DBE).

The double bond equivalents (DBE) is the number of molecules of H2 that would have to be added to a molecule to convert all pi bonds to single bonds, and all rings to acyclic structures. The DBE number can be calculated from the formula using the following equation: $DBE = nC - \left(H/2\right) + \left(N/2\right) + 1 ,$ where: nC = number of carbon atoms, H = number of hydrogen and halogen atoms, and N = number of nitrogen atoms.

The fold change is a measure that describes how much a quantity changes between an original and a subsequent measurement. Fold change normally given in terms of ratios, and, log-ratios are often used for analysis and visualization of fold changes. The logarithm to base 2 is normally used and is designated as log2(fold change) as it is easy to interpret. E.g. a doubling in the original scaling is equal to a log2(fold change) of 1, a quadrupling is equal to a log2(fold change) of 2 and so on. At the other end, the measure is symmetric when the change decreases by an equivalent amount e.g. a halving is equal to a log2(fold change) of -1, a quartering is equal to a log2(fold change) of -2 and so on.

Tandem mass spectrometry (MS/MS) involves two or more stages of mass spectrometry separated by a fragmentation step. The first mass analyzer selects and isolates ions of a specific m/z (MS1 dimension), the so-called precursor ions, which are then fragmented, for example by collision-induced dissociation (CID). The resulting fragment ions are separated based on their m/z in a second mass analyzer, and detected (MS2 dimension) to form a MS/MS spectrum. Like a regular mass spectrum, an MS/MS spectrum has peaks that represent detected ions. In a typical MS/MS spectrum, peaks representing both the (unfragmented) precursor ion, as well as fragment ions can be found. The fragment ions can be used to identify the chemical structure of the selected and isolated precursor. Trapped ion mobility spectroscopy (TIMS) involves using an electric field to hold ions stationary against a moving gas. This setup allows the drift force to be compensated by the electric field, effectively separating ion packages based on their size-to-charge ratio, commonly referred to as ion mobility or collisional cross section (CCS).

A mass-mobility heatmap is a visual representation used in ion mobility spectrometry (IMS) coupled with mass spectrometry (MS) to analyze complex samples. This heatmap plots the mass-to-charge ratio (m/z) of ions on one axis and their ion mobility on the other, creating a two-dimensional map that helps in identifying and characterizing compounds based on their mass and mobility. In practical terms, the mass-mobility heatmap allows to visualize how ions are separated not only by their mass but also by their shape and size, which affects their mobility through a gas. This is particularly useful for distinguishing between isomeric compounds (those with the same mass but different structures) and for identifying isobaric compounds with similar masses but different mobilities. A heatmap shows the intensity values of raw signals, peaks or features (e.g. a fragment of a precursor) in all of the spectra as a function of the spatial coordinates. The x-axis of the heatmap represents the mass dimension of the analysis, the y-axis represents the mobility dimension of the analysis, and the signal intensity is displayed using color coding and/or equipotential lines.

**Parallel Reaction Monitoring** (PRM) is a targeted mass spectrometry technique used for the precise quantification and characterization of specific biomolecules such as lipids, carbohydrates, proteins or peptides or combinations thereof in complex samples. In PRM, the process begins with the isolation of target precursor ions typically using a quadrupole mass filter. These isolated ions are then fragmented in a collision cell, and the resulting fragment ions are detected and quantified using a high-resolution mass analyzer, such as an Orbitrap or a time-of-flight (TOF) mass spectrometer. In contrast to DDA, PRM isolates and fragments pre-defined precursors (in the same way as SRM/MRM) but records all product ions of a pre-defined precursor.

**Data-dependent acquisition** (DDA) is another targeted mass spectrometry technique used for the precise quantification and characterization of specific biomolecules such as lipids, carbohydrates, proteins or peptides or combinations thereof in complex samples, in which precursor ions are not preselected but selected sequentially for fragmentation in order of decreasing intensity. So DDA automatically selects the most abundant precursor ions for isolation and fragmentation and monitors all product ions.

**Data-independent acquisition** (DIA) is an essentially non-targeted mass spectrometry technique used for the broad characterization of specific biomolecules such as lipids, carbohydrates, proteins or peptides or combinations thereof in complex samples, in which all ions within a selected mass-to-charge range are selected for fragmentation. This approach does not require any preliminary method design prior to the sample injection. Since the LC-MS acquisition covers the complete analyte contents of a sample through the entire mass and retention time (RT) ranges the data can be mined a posteriori for any analyte, so e.g. lipid/precursor of peptide/precursor of interest. Data is acquired in a data independent manner, on the complete mass range (e.g. 200-2000 Thomson) and through the entire chromatography, disregarding of the content of the sample.

**Parallel Accumulation SErial Fragmentation** (PASEF) leverages trapped ion mobility spectrometry (TIMS) to accumulate ions in parallel before fragmenting them in series. This process involves holding ions stationary in a tunnel using opposing forces of gas flow and an electric field. The ions find an equilibrium point based on their collisional cross-section, which is a function of their size and shape. Once the desired number of ions have been accumulated, they are eluted by gradually lowering the electric field strength, allowing them to enter the mass spectrometer for fragmentation. This method improves the signal-to-noise ratio by concentrating ions of the same mass-to-charge ratio, which enhances the detection of low-abundance species.

In the proposed method, the first and second sample may differ in origin, including from healthy and diseased patients, differ in treatment/perturbation, including a first sample without treatment/perturbation and a second sample having been subjected to treatment/perturbation, including exposure to an external influence (such as chemical influence, including medicaments or the like), physical influence, including irradiation or the like, but also aging, and combinations thereof. The first and second sample may differ in underlying genetic material, including comparing wild type and genetically modified, in particular knockout gene-based materials.

According to a first preferred embodiment of the present invention, the analytes are lipids, and the homologous row is defined as a function of double bond equivalents in the sidechains, or as a function of the carbon atoms in the sidechain. Preferably the homologous row is defined as the same number of double bond equivalents with changing number of carbon atoms in the sidechain or is defined as the same number of carbon atoms in the sidechains with changing number of double bond equivalents.

The analytes can also be glycosylated systems, then the homologous row is defined as a function of number and/or type of sugar moieties.

In step iii) and in calculating for each analyte matrices of structural features in step iv) preferably the binary logarithm of the ratio is used.

According to yet another preferred embodiment, in step iv), in particular in lipidomics, for identifying analytes with differential behavior can be implemented by using a Kendrick mass defect (KMD or SKMD) representation of said change in intensity data and/or
by calculating for each analyte matrices of structural features characterizing the respective homologous row by using the change of the abundance of the respective structural feature, preferably as a ratio, most preferably as the binary logarithm of such a ratio.

For the case of analysis of homologous rows of lipids, a Kendrick mass defect plot in terms of CH2 units is particularly helpful to sort and correspondingly visualize the homologous rows. This as in this representation for example as a function of retention time, systems of different length, so different number of CH2 units, will appear for the same value of the Kendrick mass defect. So, if for example in a representation where on the X axis the retention time is given and on the Y axis the Kendrick mass defect, lipids having sidechains of different numbers of CH2 units, so different side chain length will appear at the same height but at different RT positions. On the other hand, in the same representation systems having different double bond equivalents due to having different KMD values will appear at different height. So, a Kendrick mass defect plot is one useful possibility to sort and analyze lipids in the proposed way in terms of DBE homologous rows and at the same time in terms of chain length homologous rows.

In case of glycosylated systems, a similar approach is possible, in this case the Kendrick mass defect plot can e.g. be expressed in terms of sugar units or the like.

Preferably, in the Kendrick mass defect representation differential behavior is visualized along homologous rows.

The matrices in step iv) may include at least one dimension characterizing the homologous row, preferably two dimensions each characterizing a homologous row of the same structural element of the analyte, in a different way.

Preferably in lipidomics the matrix has one dimension given by binary logarithm intensity fold change and at least one, preferably two further dimensions,
one further dimension given by the lipids sidechain double bond equivalents or the number of lipids sidechain double bonds and/or
one further dimension given by the total lipids sidechain chain length or the number of lipids sidechain carbon atoms.

Preferably the matrix is a three-dimensional matrix with chain length, double bond and intensity fold change axes.

Normally, said untargeted LC MS/MS and said targeted LC MS/MS are methods of liquid chromatography (LC) coupled combined ion mobility and mass spectroscopy analysis, preferably comprising the following steps:
introducing precursor analyte ions into an ion mobility separator (IMS), preferably a trapped ion mobility spectrometry (TIMS) separator,
separating the precursor analyte ions according to mobility in the ion mobility separator, sequentially releasing precursor analyte ions from said ion mobility separator according to their ion mobility,
introducing said released precursor analyte ions into a mass filter which selectively transmits precursor analyte ions having m/z values falling within a controllable mass window,
fragmenting the precursor analyte ions transmitted through said mass filter to generate fragment ions,
carrying out a mass spectroscopy measurement on said fragment ions, wherein each fragment ion is associated with a mass window and an ion mobility (IM) range, and associating detected fragments with its corresponding precursor ion,
wherein preferably said second ion mobility separator and said mass filter are controlled in a synchronized manner preferably such as to carry out a plurality of ion mobility (IM) scans, during which precursor ions of increasing or decreasing IM are successively released from said second ion mobility separator (IMS), and during which the mass window of said mass filter is shifted continuously or stepwisely towards lower or higher m/z values, respectively, and wherein preferably said step of associating a detected fragment with its corresponding precursor ion is based on determining or utilizing the corresponding mass windows and ion mobility (IM) ranges associated with various occurrences of said fragment in said mass spectrometry measurement.

Said untargeted LC MS/MS and said targeted LC MS/MS are preferably methods of liquid chromatography (LC) coupled combined ion mobility and mass spectroscopy analysis with an ion mobility separator (IMS), preferably a trapped ion mobility spectrometry (TIMS) separator, and said untargeted LC MS/MS is a DIA PASEF measurement and/or said targeted LC MS/MS is a prm PASEF measurement, the scheduling of which is determined by the analytes established be of interest in step iv).

Said ion mobility separator (IMS) is normally a TIMS analyzer with parallel accumulation and separation,
in which the precursor ions are accumulated in an accumulation section in an RF ion trap, in which the ions are radially confined by an RF field and pushed by a gas flow against a rising edge of an axial electric DC field barrier such that the transferred ions are spatially separated along the rising edge according to ion mobility, and wherein the height of the electric DC field barrier is chosen such as to retain only ions having an ion mobility higher than a threshold value
followed by transferring at least a subset of the accumulated ions into a trapping ion mobility separator in a separation section, radially confined by an RF field and pushed by a gas flow against a rising edge of an axial electric DC field barrier such that the transferred ions are spatially separated along the rising edge according to ion mobility, and wherein the height of the electric DC field barrier at the beginning of the release is preferably chosen such as to retain only ions having an ion mobility higher than said threshold value.

Said ion mobility separator (IMS) and said mass filter are normally scheduled in a synchronized manner such as to carry out a plurality of ion mobility (IM) scans, during which precursor ions of increasing or decreasing IM are successively released from said second ion mobility separator (IMS), and during which the mass window of said mass filter is shifted continuously or stepwisely towards lower or higher m/z values, respectively,
wherein in said IM scans, adjacent mass windows that are associated with consecutive mass spectroscopy measurements of fragment ions overlap, such that the precursor ions transmitted through said mass filter during one IM scan are located in at least one continuous scan region in an m/z-IM plane which extends in a generally diagonal direction in said m/z-IM plane,
wherein adjacent scan regions associated with different IM scans overlap in the m/z-direction.

In said IM scans, adjacent mass windows that are associated with consecutive mass spectroscopy measurements of fragment ions may overlap by at least 30% of their width, and/or wherein adjacent scan regions associated with different IM scans overlap in the m/z-direction by at least 33% of their width in m/z direction.

Preferably, step i) entails preprocessing of the raw data in an untargeted fashion yielding a feature table, wherein each feature comprises analytes and respective isotope patterns, aligned across said first and second sample.

Furthermore, the present invention relates to the use of such a method for the analysis of two differential complex samples, including for the comparison of at least two different samples in lipidomics, proteomics and metabolomics.

Also, the present invention relates to an apparatus for combined liquid chromatography (LC), ion mobility and mass spectroscopy analysis, for carrying out the method according to any of the preceding claims, said apparatus preferably comprising:
a liquid chromatography (LC) unit,
an ionization unit ionizing precursors eluting as a function of time from said liquid chromatography unit,
an ion mobility separator (IMS) in the form of at least one trapped ion mobility spectrometry (TIMS) separator for receiving and sequentially releasing precursor ions according to their ion mobility,
a mass filter arranged to receive said released precursor ions and to selectively transmit precursor ions having m/z values falling within a controllable mass window,
a fragmentation device for fragmenting the precursor ions transmitted through said mass filter to generate fragment ions,
an apparatus for carrying out a mass spectroscopy measurement on said fragment ions, wherein each fragment ion is associated with a mass window and an ion mobility (IM) range, and a control system,
wherein said control system is configured to control said IMS and said mass filter for the targeted LC MS/MS of the first and/or the second sample in a synchronized manner for the scheduling of the frames, by selecting precursors on the basis of the analysis in step iv. Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a Kendrick Mass Defect Plot of CH2 vs. Retention Time; shading/color-coding and bubble size reflects log2(fold change) between two conditions (KO vs. WT);
- Fig. 2: shows a Kendrick Mass Defect Plot of CH2 vs. Retention Time; color-coding by annotated lipid classes; bubble size reflects log2(fold change) between two conditions (KO vs. WT);
- Fig. 3: shows a Kendrick Mass Defect Plot of CH2 vs. Retention Time; shading/color-coding and bubble size reflects log2(fold change) between two conditions (KO vs. WT); zoomed into one lipid class, here triglycerides;
- Fig. 4: shows a Kendrick Mass Defect Plot of CH2 vs. Retention Time; shading/color-coding and bubble size reflects log2(fold change) between two conditions (KO vs. WT); zoomed into one lipid class, here triglycerides, and annotated;
- Fig. 5: shows a comparison of the fold change grid perspective (left) and the single lipid comparison (right), wherein the single selected lipid is marked with a rectangle in the left plot, the left rectangle indicating the knock-out (KO) sample measurement and the right rectangle indicating the wild-type sample measurement (WT), and wherein in a) the triglyceride TG 16:0_18:0_18:0 is given and in b) TG 16:0_18:0_20:1;

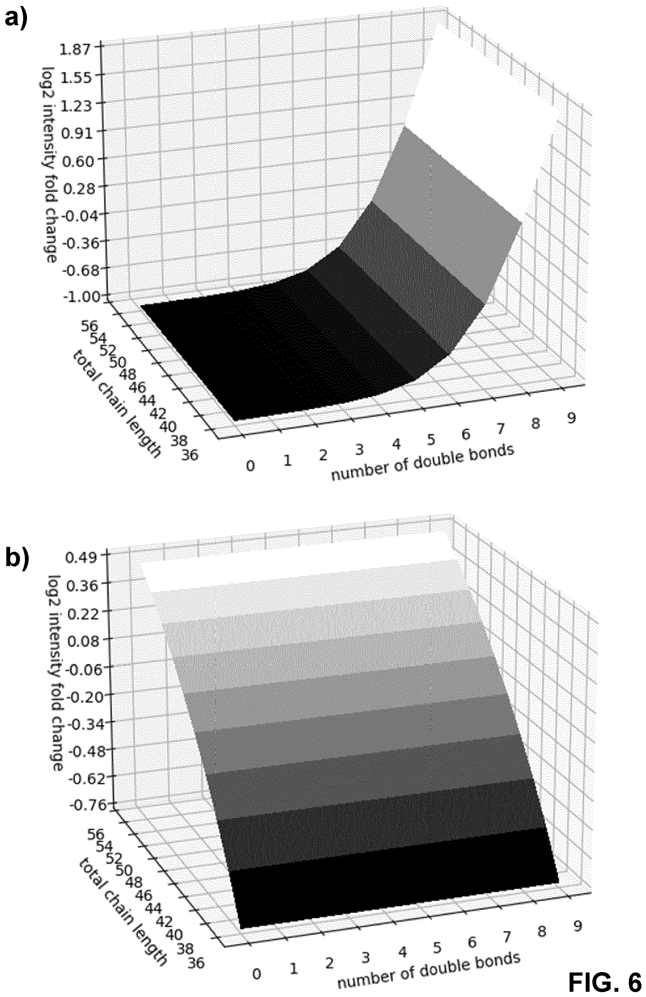

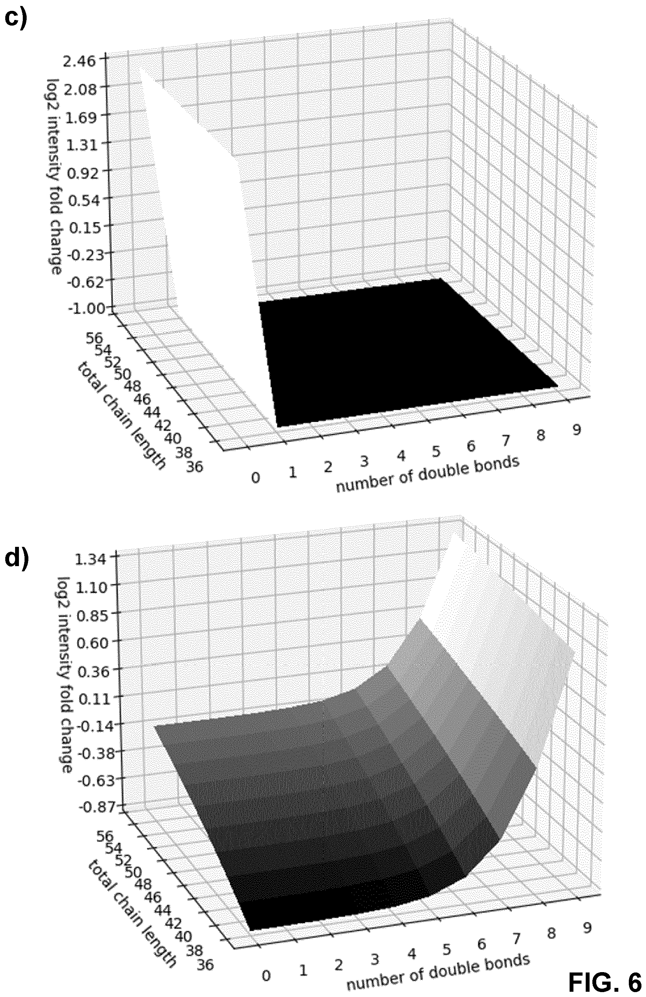

- Fig. 6: shows in a) the log2(fold change) of lipid species in sample group A over sample group B; a possible biological interpretation is, that sample group A has a higher tendency to form double bonds; in b) the log2(fold change) of lipid species in sample group A over sample group B; a possible biological interpretation is, that sample group A has a higher tendency towards forming longer side chains; in c) the log2(fold change) of lipid species in sample group A over sample group B; a possible biological interpretation is, that sample group A is incapable of desaturating this lipid class; in d) the log2(fold change) of lipid species in sample group A over sample group B; a possible biological interpretation is, that sample group A has a higher tendency to longer side chains with more double bonds.

### DESCRIPTION OF PREFERRED EMBODIMENTS

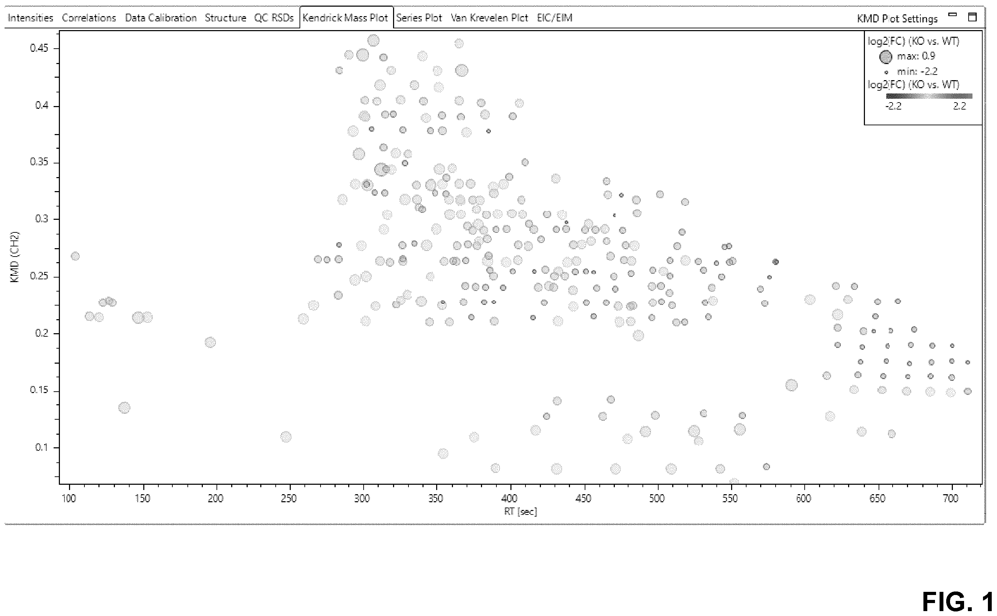

Fig. 1 shows a Kendrick Mass Defect Plot of CH₂ (KMD CH2) vs. Retention Time (RT) of a pair of lipidomics samples comparing a wild type (WT) sample and a knockout (KO) sample. Shading/color-coding and bubble size reflects log2(fold change) between the two conditions in the sampes (KO vs. WT). So, this is basically the result of the above-mentioned step i) but already including the differential calculation of step iii) for the bubble size in a specific KMD representation after having collected the raw data in the untargeted LC MS/MS experiments carried out on each of the samples. One can already recognize horizontal rows in this representation suggesting homologous rows of corresponding analytes.

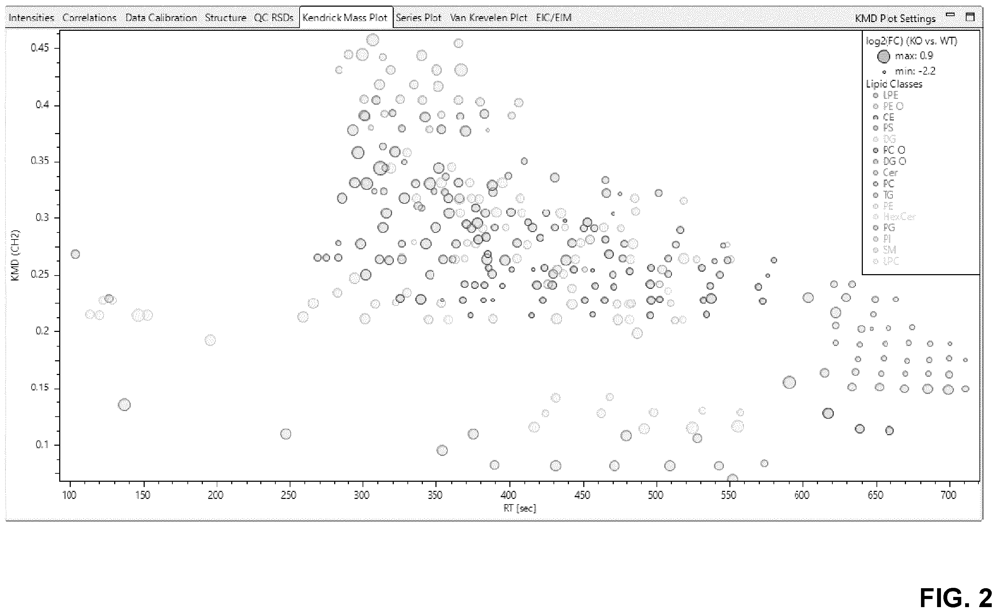

Fig. 2 shows a Kendrick Mass Defect Plot of CH2 vs. Retention Time like the one illustrated in Fig. 1, again with bubble size reflecting the log2(fold change) between two conditions of the samples (KO vs. WT). However, in this case shading/color-coding reflects the annotation of the lipid classes. So, this is basically the result of the above-mentioned step ii) but already including the differential calculation of step iii) for the bubble size. Annotation is typically either carried out using rule-based annotation interpreting one or more of MS1, MS2, isotope pattern, RT and CCS, or by recurring to a spectral library containing ion mobility, precursor and fragment information and using that information for annotating the precursors based on ion mobility, MS1 and MS2 as measured. Standard lipid abbreviations are used in the legend on the margin, the further illustrations are focusing on the group of triglycerides, abbreviated with TG.

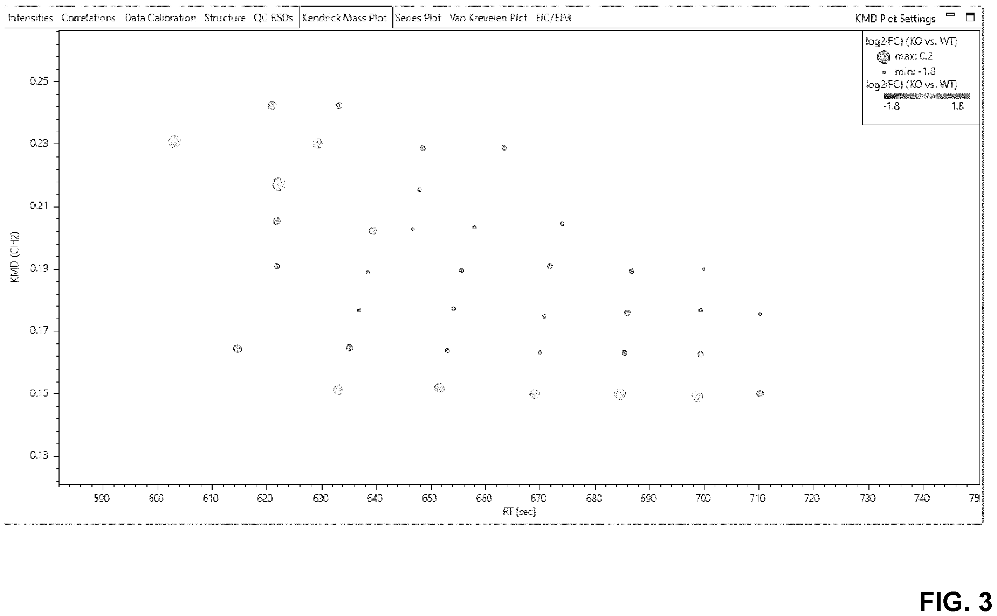

**Fig. 3** is zooming into the area of the triglycerides, which in Fig. 2 are appearing right below the legend box on the bottom right. The figure shows a Kendrick Mass Defect Plot of CH2 vs. Retention Time, again shading/color-coding and bubble size reflects log2(fold change) between two conditions (KO vs. WT).

In such a representation, lipids which differ only by the length of the sidechain but not by their saturation will appear on horizontal lines because they have the same Kendrick mass defect value. Typically, longer sidechain systems are to the right in this representation, but this depends on the liquid chromatography system.

In such a representation, lipids which differ by the degree of saturation in the sidechain, so in terms of DBE values, will appear at different heights. Systems which differ only by the DBE value but do not differ in chain length will therefore appear along vertical lines.

So, the Kendrick mass plot representation nicely provides for a sorting of the analytes in terms of two different types of homologous rows, namely in terms of length and DBE values, in two dimensions. In this plot, one can see, that saturated lipids (no double bonds; they constitute the bottom horizontal homologous series) appear with similar intensities in both WT and KO samples. Lipids with one or more double bounds however (the other horizontal homologous series) appear with lower intensities in the KO samples. This may be interpreted as a lower tendency to form unsaturated lipids in the KO mutants. This is a view, that neither the fold-change of a single lipid species, nor the fold-change within the entire lipid class would have provided.

How this can now be used for identifying analytes with differential behavior within homologous rows in accordance with step iv) above is again illustrated in the following figure.

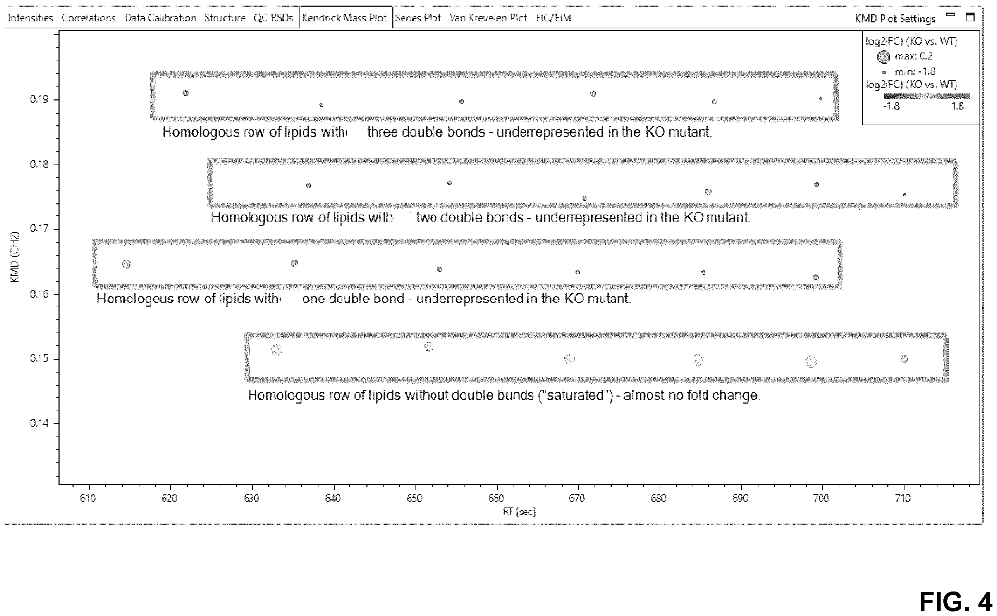

**Fig. 4** shows a Kendrick Mass Defect Plot of CH2 vs. Retention Time, wherein again shading/color-coding and bubble size reflects log2(fold change) between two conditions (KO vs. WT). In this case the side chain length homologous series are indicated by rectangular boxes and annotated. The figure thus by comparing the intensity patterns of sidechain length homologous rows for different saturation degrees (rectangular boxes at different heights) this reveals that this is the above-mentioned example, in which a mutant strain of an organism is uncapable of (or hampered in) desaturating (i.e. introducing DBEs) lipid species of a certain lipid class. This does not change the total lipid signal intensity in this class, but it will change the topology of the above-described pattern, as one can see from this figure.

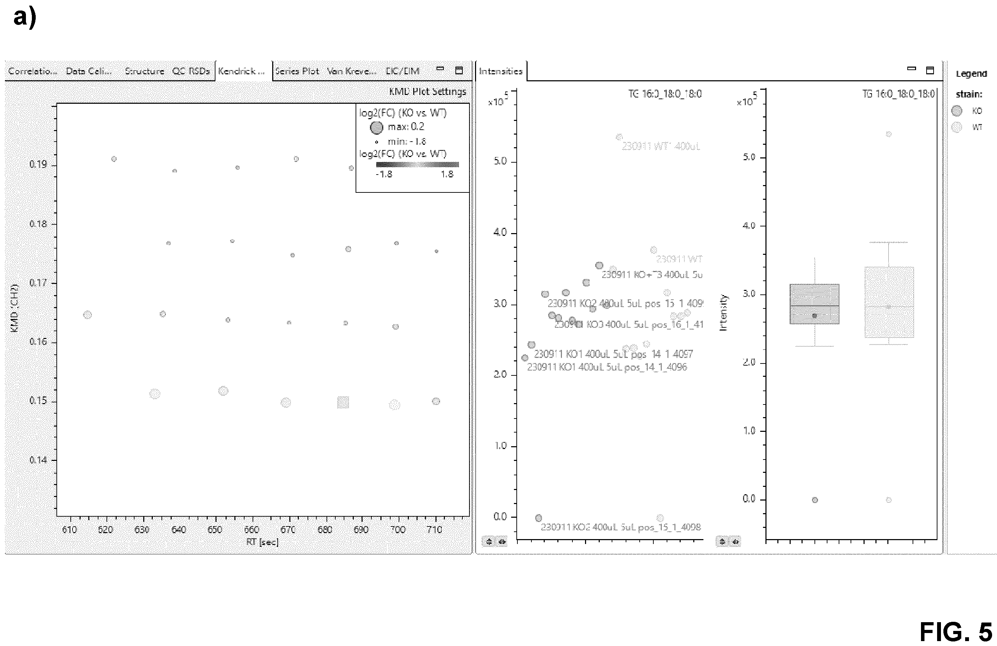

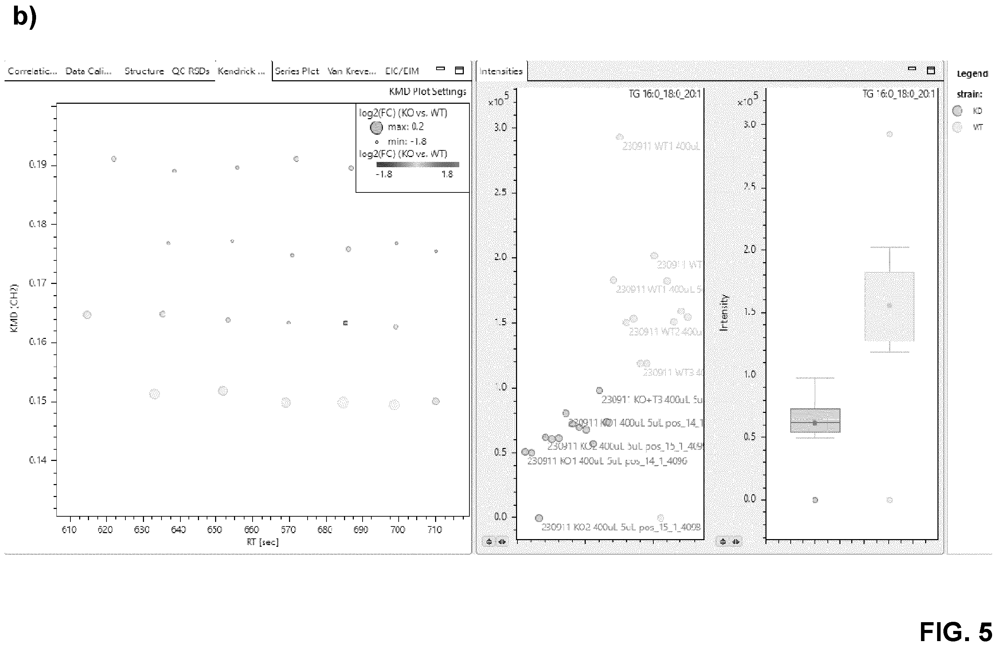

Going one level further down in detail looking at specific analytes within one class and homologous row, **Fig. 5** shows a comparison of the fold change grid perspective (left) and the single lipid comparison (right), wherein the single selected lipid is marked with a rectangle in the left plot, the left rectangle in the rightmost frame indicating the knock-out (KO) sample measurement and the right rectangle in the rightmost frame indicating the wild-type sample measurement (WT). In a) this is given for the triglyceride TG 16:0_18:0_18:0 and in b) for TG 16:0_18:0_20:1. Again one can see here that the wild type intensity and the knockout intensity are essentially the same for the systems with no double bonds (a), while there is a significantly lower intensity for the wild type knockout compared with the wild type (b). While the box plots on the right side of the figures allow to visualize this change for individual molecular lipid species, the embedding into homologous series puts it into context with further members of the lipid class and reveals systematic changes in dependency of the number of carbon atoms in the side chains (nC) and double bond equivalents (DBE).

**Fig. 6** illustrates different situations for the matrix representation. In sample groups A and B, distinct intensity distributions across lipid species within a specific lipid class are observed. Although the mean intensity of lipid species might be similar between the two groups, examining the fold changes of individual lipid species can provide more insightful information. By interpreting these fold changes as a surface on a grid defined by carbon chain length and double bonds, systematic variations across the groups can be identified. The approach preserves the relationships in terms of lipid class, chain length, and double bonds, while also considering individual lipid species, unlike methods that focus solely on the total or mean abundance of a lipid class.

Fig. 6 in a) shows a stereotypic example of the log2(fold change) of lipid species in sample group A over sample group B. A possible biological interpretation of this is, that sample group A has a higher tendency to form double bonds in that lipid class, compared to group B.

Fig. 6 in b) shows a stereotypic example of the log2(fold change) of lipid species in sample group A over sample group B. A possible biological interpretation is, that sample group A has a higher tendency towards forming longer side chains in that lipid class, compared to group B.

Fig. 6 in c) shows a stereotypic example of the log2(fold change) of lipid species in sample group A over sample group B. A possible biological interpretation is, that sample group A is incapable of desaturating this lipid class in that lipid class.

Fig. 6 in d) shows a stereotypic example of the log2(fold change) of lipid species in sample group A over sample group B. A possible biological interpretation is, that sample group A has a higher tendency to longer side chains with more double bonds in that lipid class, compared to group B.

## Claims

1. Method for the identification of molecular changes between homologous rows of analytes of a first sample and of a second sample different from the first sample, both samples analyzed by using untargeted LC MS/MS experiments, wherein said homologous rows of analytes systematically differ by at least one repeating structural feature,
wherein the method comprises the following steps:
i) identifying analytes in the data obtained from the first sample and in the data obtained from the second sample;
ii) annotating said analytes in the data obtained from the first sample and in the data obtained from the second sample, preferably by using a spectral library, rule-based annotation or simulations, or a combination thereof;
iii) calculating the change of intensity of each annotated analyte between the two samples, preferably as a ratio, most preferably as the binary logarithm of such a ratio;
iv) sorting the data of the analytes as a function of said at least one repeating structural feature and thereby identifying analytes with differential intensity behavior within homologous rows of analytes in said first sample and said second sample;
v) scheduling a targeted LC MS/MS of the first and/or the second sample for the identified analytes.

2. Method according to claim 1, wherein the first and second sample are biological samples, preferably of human origin, in a proteomics or lipidomics or metabolomics experiment.

3. Method according to any of the preceding claims, wherein the first and second sample differ in origin, including from healthy and diseased patients, in treatment, including a first sample without treatment and a second sample having been subjected to treatment, including exposure to an external influence, in particular chemical influence, physical influence, aging, and combinations thereof, in underlying genetic material, including comparing wild type and genetically modified, in particular knockout gene-based materials.

4. Method according to any of the preceding claims, wherein the analytes are lipids, and wherein the homologous row is defined as a function of double bond equivalents in the sidechains, and/or as a function of the carbon atoms in the sidechain, preferably the homologous row being defined as the same number of double bond equivalents with changing number of carbon atoms in the sidechain or being defined as the same number of carbon atoms in the sidechains with changing number of double bond equivalents
or wherein the analytes are glycosylated systems, and wherein the homologous row is defined as a function of number and/or type of sugar moieties.

5. Method according to any of the preceding claims, wherein in step iii) and in calculating for each analyte matrices of structural features in step iv) the binary logarithm of the ratio is used.

6. Method according to any of the preceding claims, wherein in step iv), in particular in lipidomics, analytes with differential behavior within homologous rows of analytes in said first sample and said second sample are identified
by using a Kendrick mass defect representation of said change in intensity data and/or
by calculating for each analyte matrices of structural features characterizing the respective homologous row by using the change of the abundance of the respective structural feature, preferably as a ratio, most preferably as the binary logarithm of such a ratio,
wherein preferably in the Kendrick mass defect representation differential behavior is visualized along homologous rows,
or wherein preferably the matrices in step iv) include at least one dimension characterizing the homologous row, preferably two dimensions each characterizing a homologous row of the same structural element of the analyte, in a different way, wherein preferably in lipidomics the matrix has one dimension given by binary logarithm intensity fold change and at least one, preferably two further dimensions,
one further dimension given by the lipids sidechain double bond equivalents or the number of lipids sidechain double bonds and/or
one further dimension given by the total lipids sidechain chain length or the number of lipids sidechain carbon atoms,
preferably the matrix being a three-dimensional matrix with chain length, double bond and intensity fold change axes.

7. Method according to any of the preceding claims, wherein said untargeted LC MS/MS and said targeted LC MS/MS are methods of liquid chromatography (LC) coupled combined ion mobility and mass spectroscopy analysis,
preferably comprising the following steps:
introducing precursor analyte ions into an ion mobility separator (IMS), preferably a trapped ion mobility spectrometry (TIMS) separator,
separating the precursor analyte ions according to mobility in the ion mobility separator,
sequentially releasing precursor analyte ions from said ion mobility separator according to their ion mobility,
introducing said released precursor analyte ions into a mass filter which selectively transmits precursor analyte ions having m/z values falling within a controllable mass window,
fragmenting the precursor analyte ions transmitted through said mass filter to generate fragment ions,
carrying out a mass spectroscopy measurement on said fragment ions, wherein each fragment ion is associated with a mass window and an ion mobility (IM) range, and associating detected fragments with its corresponding precursor ion,
wherein preferably said second ion mobility separator and said mass filter are controlled in a synchronized manner preferably such as to carry out a plurality of ion mobility (IM) scans, during which precursor ions of increasing or decreasing IM are successively released from said second ion mobility separator (IMS), and during which the mass window of said mass filter is shifted continuously or stepwisely towards lower or higher m/z values, respectively,
and wherein preferably said step of associating a detected fragment with its corresponding precursor ion is based on determining or utilizing the corresponding mass windows and ion mobility (IM) ranges associated with various occurrences of said fragment in said mass spectrometry measurement.

8. Method according to any of the preceding claims, wherein said untargeted LC MS/MS and said targeted LC MS/MS are methods of liquid chromatography (LC) coupled combined ion mobility and mass spectroscopy analysis with an ion mobility separator (IMS), preferably a trapped ion mobility spectrometry (TIMS) separator, and wherein said untargeted LC MS/MS is a DIA PASEF measurement and/or wherein said targeted LC MS/MS is a prm PASEF measurement, the scheduling of which is determined by the analytes established be of interest in step iv).

9. Method according to any of the preceding claims 7 or 8, wherein said ion mobility separator (IMS) is a TIMS analyzer with parallel accumulation and separation,
in which the precursor ions are accumulated in an accumulation section in an RF ion trap, in which the ions are radially confined by an RF field and pushed by a gas flow against a rising edge of an axial electric DC field barrier such that the transferred ions are spatially separated along the rising edge according to ion mobility, and wherein the height of the electric DC field barrier is chosen such as to retain only ions having an ion mobility higher than a threshold value
followed by transferring at least a subset of the accumulated ions into a trapping ion mobility separator in a separation section, radially confined by an RF field and pushed by a gas flow against a rising edge of an axial electric DC field barrier such that the transferred ions are spatially separated along the rising edge according to ion mobility, and wherein the height of the electric DC field barrier at the beginning of the release is preferably chosen such as to retain only ions having an ion mobility higher than said threshold value.

10. Method according to any of the preceding claims 7 - 9, wherein said ion mobility separator (IMS) and said mass filter are scheduled in a synchronized manner such as to carry out a plurality of ion mobility (IM) scans, during which precursor ions of increasing or decreasing IM are successively released from said second ion mobility separator (IMS), and during which the mass window of said mass filter is shifted continuously or stepwisely towards lower or higher m/z values, respectively,
wherein in said IM scans, adjacent mass windows that are associated with consecutive mass spectroscopy measurements of fragment ions overlap, such that the precursor ions transmitted through said mass filter during one IM scan are located in at least one continuous scan region in an m/z-IM plane which extends in a generally diagonal direction in said m/z-IM plane,
wherein adjacent scan regions associated with different IM scans overlap in the m/z-direction.

11. Method according to claim 10, wherein in said IM scans, adjacent mass windows that are associated with consecutive mass spectroscopy measurements of fragment ions overlap by at least 30% of their width, and/or wherein adjacent scan regions associated with different IM scans overlap in the m/z-direction by at least 33% of their width in m/z direction.

12. Method according to any of the preceding claims, wherein step i) entails preprocessing of the raw data in an untargeted fashion yielding a feature table, wherein each feature comprises analytes and respective isotope patterns, aligned across said first and second sample.

13. Use of the method of any of the preceding claims for the analysis of two differential complex samples, including samples in lipidomics, proteomics and metabolomics.

14. An apparatus for combined liquid chromatography (LC), ion mobility and mass spectroscopy analysis, for carrying out the method according to any of the preceding claims, said apparatus preferably comprising:
a liquid chromatography (LC) unit,
an ionization unit ionizing precursors eluting as a function of time from said liquid chromatography unit,
an ion mobility separator (IMS) in the form of at least one trapped ion mobility spectrometry (TIMS) separator for receiving and sequentially releasing precursor ions according to their ion mobility,
a mass filter arranged to receive said released precursor ions and to selectively transmit precursor ions having m/z values falling within a controllable mass window,
a fragmentation device for fragmenting the precursor ions transmitted through said mass filter to generate fragment ions,
an apparatus for carrying out a mass spectroscopy measurement on said fragment ions,
wherein each fragment ion is associated with a mass window and an ion mobility (IM) range, and a control system,
wherein said control system is configured to control said IMS and said mass filter for the targeted LC MS/MS of the first and/or the second sample in a synchronized manner for the scheduling of the frames, by selecting precursors on the basis of the analysis in step iv.

15. An apparatus according to claim 14, wherein the trapped ion mobility spectrometry (TIMS) separator is a TIMS analyzer with parallel accumulation and separation, in particular operating using a method comprising the steps:
(a) accumulating ions from the first ion mobility separator in an RF ion trap;
(b) transferring at least a subset of the accumulated ions into a trapping ion mobility separator, in which the transferred ions are radially confined by an RF field and pushed by a gas flow against a rising edge of an axial electric DC field barrier such that the transferred ions are spatially separated along the rising edge according to ion mobility;
(c) successively releasing the transferred ions according to their ion mobility by decreasing the height of the electric DC field barrier while ions from the ion source are further accumulated in the RF ion trap; and
(d) restoring the height of the electric DC field barrier which triggers a consecutive transfer of the accumulated ions from the RF ion trap into the trapping ion mobility separator.
